(19) Europäisches Patentamt
European Patent Office
Office européen des brevets

(11) **EP 1 550 720 B1**

(12) **EUROPEAN PATENT SPECIFICATION**

(45) Date of publication and mention
of the grant of the patent:
**31.03.2010 Bulletin 2010/13**

(21) Application number: **03730778.2**

(22) Date of filing: **02.06.2003**

(51) Int Cl.:
***C07K 14/72*** *(2006.01)*

(86) International application number:
**PCT/JP2003/006942**

(87) International publication number:
**WO 2003/102188 (11.12.2003 Gazette 2003/50)**

(54) **MUTATED ANDROGEN RECEPTOR, CANCER CELLS EXPRESSING THE SAME, METHOD OF CONSTRUCTING THE SAME AND USE THEREOF**

MUTIERTER ANDROGENREZEPTOR, DIESEN EXPRIMIERENDE KREBSZELLEN, VERFAHREN ZUR KONSTRUKTION DAVON UND VERWENDUNG DAVON

RECEPTEUR D'ANDROGENE MUTE, CELLULES CANCEREUSES L'EXPRIMANT, PROCEDE DE CONSTRUCTION DE CE RECEPTEUR ET UTILISATIONS CORRESPONDANTES

(84) Designated Contracting States:
**AT BE BG CH CY CZ DE DK EE ES FI FR GB GR HU IE IT LI LU MC NL PT RO SE SI SK TR**

(30) Priority: **03.06.2002 JP 2002162206**
**30.08.2002 JP 2002255612**

(43) Date of publication of application:
**06.07.2005 Bulletin 2005/27**

(60) Divisional application:
**09075054.8 / 2 090 656**

(73) Proprietor: **Takeda Pharmaceutical Company Limited**
**Osaka-shi,**
**Osaka 541-0045 (JP)**

(72) Inventors:
• **HARA, Takahito**
**Osaka-shi, Osaka 532-0013 (JP)**
• **KUSAKA, Masami**
**Kobe-shi, Hyogo 651-2102 (JP)**
• **MIYAZAKI, Junichi**
**Kobe-shi, Hyogo 658-0073 (JP)**

(74) Representative: **Böhm, Brigitte et al**
**Weickmann & Weickmann**
**Patentanwälte**
**Postfach 86 08 20**
**81635 München (DE)**

(56) References cited:
**EP-A- 1 466 902     WO-A-01/66599**

**US-B1- 6 291 194**

• ZHAO ET AL: "TWO MUTATIONS IDENTIFIED IN THE ANDROGEN RECEPTOR OF THE NEW HUMAN PROSTATE CANCER CELL LINE MDA PCA 2A" JOURNAL OF UROLOGY, BALTIMORE, MD, US, vol. 162, no. 11, December 1999 (1999-12), pages 2192-2199, XP005376218 ISSN: 0022-5347
• BENTEL J M ET AL: "ANDROGEN RECEPTOR EXPRESSION IN PRIMARY PROSTATE CANCERS OF LOBUND-WISTAR RATS AND IN TUMOR-DERIVED CELL LINES" IN VITRO CELLULAR & DEVELOPMENTAL BIOLOGY. ANIMAL, TISSUE CULTURE ASSOCIATION, COLUMBIA, MD, US, vol. 35, no. 10, November 1999 (1999-11), pages 655-662, XP009026700 ISSN: 1071-2690
• KOKONTIS J M ET AL: "PROGRESSION OF LNCAP PROSTATE TUMOR GELLS DURING ANDROGEN DEPRIVATION: HORMONE-INDEPENDENT GROWTH, REPRESSION OF PROLIFERATION BY ANDROGEN, AND ROLE FOR P27KIP1 IN ANDROGEN-INDUCED CELL CYCLE ARREST" MOLECULAR ENDOCRINOLOGY, BALTIMORE, MD, US, vol. 12, 1998, pages 941-953, XP002916571 ISSN: 0888-8809

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- VELDSCHOLTE J ET AL: "A mutation in the ligand binding domain of the androgen receptor of human LNCaP cells affects steroid binding characteristics and response to anti-androgens" BIOCHEMICAL AND BIOPHYSICAL RESEARCH COMMUNICATIONS, ACADEMIC PRESS INC. ORLANDO, FL, US, vol. 173, no. 2, 14 December 1990 (1990-12-14), pages 534-540, XP002972371 ISSN: 0006-291X
- HARA TAKAHITO ET AL: "Novel mutations of androgen receptor: a possible mechanism of bicalutamide withdrawal syndrome." CANCER RESEARCH. 1 JAN 2003, vol. 63, no. 1, 1 January 2003 (2003-01-01), pages 149-153, XP002382326 ISSN: 0008-5472
- HAAPALA K ET AL: "Androgen receptor alterations in prostate cancer relapsed during a combined androgen blockade by orchiectomy and bicalutamide." LABORATORY INVESTIGATION; A JOURNAL OF TECHNICAL METHODS AND PATHOLOGY. DEC 2001, vol. 81, no. 12, December 2001 (2001-12), pages 1647-1651, XP002382327 ISSN: 0023-6837
- GOTTLIEB BRUCE ET AL: "Update of the androgen receptor gene mutations database" HUMAN MUTATION, WILEY-LISS, NEW YORK, NY, US, vol. 14, no. 2, 1999, pages 103-114, XP002173777 ISSN: 1059-7794
- SADAR M.D. ET AL.: 'Androgen-independent induction of prostate-specific antigen gene expression via cross-talk between the androgen receptor and protein kinase A signal transduction pathways' J. BIOL. CHEM. vol. 274, no. 12, 1999, pages 7777 - 7783, XP002130097
- VELDSCHOLTE J. ET AL.: 'A mutation in the ligand binding domain of the androgen receptor of human LNCaP cells affects steroid binding characteristics and response to anti-androgens' BIOCHEM. BIOPHYS. RES. COMMUN. vol. 173, no. 2, 1990, pages 534 - 540, XP002972371
- TAPLIN M.E. ET AL.: 'Selection for androgen receptor mutations in prostate cancers treated with androgen antagonist' CANCER RES. vol. 59, no. 11, 1999, pages 2511 - 2515, XP002191550
- MARCELLI M. ET AL.: 'Amino acid substitutions in the hormone-binding domain of the human androgen receptor alter the stability of the hormone receptor complex' J. CLIN. INVEST. vol. 94, no. 4, 1994, pages 1642 - 1650, XP002972372
- GADDIPATI J.P. ET AL.: 'Frequent detection of codon 877 mutation in the androgen receptor gene in advanced prostate cancers' CANCER RES. vol. 54, no. 11, 1994, pages 2861 - 2864, XP002972373

**Description**

Technical Field

The present invention relates to a mutant androgen receptor derived from human prostate cancer cells, a human prostate cancer cell line that expresses it, a method of producing them, a method of screening an antiandrogen drug that exhibits proliferation-suppressing action even on prostate cancer in the androgen-independent stage using them and the like.

Background Art

For advanced prostate cancer with metastases, it is standard practice worldwide to conduct androgen suppression endocrine therapy as the first choice. Also, in localized prostate cancer without metastases, it was demonstrated that prostate cancer progression is delayed by conducting an endocrine therapy with an antiandrogen drug after radical prostatectomy or radiotherapy; endocrine therapy is spreading for localized prostate cancer as well.

Androgens generically refer to steroid hormones possessing male sex hormone actions; testosterone, which is synthesized in the testis, dehydroepiandrosterone and androstenedione, which are synthesized in the adrenal cortex, and the like can be mentioned. Because about 95% of androgens are secreted from the testis, surgical (orchiectomy) or chemical (estrogen, LHRH agonist and the like) castration for blocking testis-derived testosterone is frequently used as androgen suppression endocrine therapy. However, because the adrenal-cortex-derived testosterone precursor is converted to highly active dihydrotestosterone (DHT) in prostate cells, it is sometimes not negligible as a proliferative stimulation to androgen-hypersensitive cancer cells, which are considered to exist at a given ratio in prostate cancer lesions, even if their abundance is apparently only several percent. For this reason, MAB (Maximal Androgen Blockade) therapy, in which an antiandrogen drug (particularly a non-steroidal drug) that antagonistically inhibits the binding of androgens to their receptors in the prostate is used in combination with castration surgery, is also conducted.

On the other hand, no effective prophylactic/therapeutic method for androgen-independent relapsed cancers, which are experienced sooner or later in almost all patients in the advanced stage undergoing endocrine therapy, has been developed. Although the mechanism of cancer relapse to endocrine therapy has not been elucidated, a plurality of factors, such as development/increase of cells that have acquired androgen hypersensitivity as a result of androgen receptor (AR) gene amplification or overexpression, and non-androgen substances (for example, glucocorticoids and antiandrogen drugs themselves) becoming acting as agonists due to AR mutations, are suggested.

For example, some clinical findings have been reported concerning the relationship between cancer relapse after antiandrogen drug administration and AR mutations. That is, AR mutations were observed in 5 of 17 patients who experienced relapsed prostate cancer after an endocrine therapy with a combination of flutamide and castration, all of which mutations were missense mutations of the 877th amino acid (corresponding to amino acid number 882 in the amino acid sequence of SEQ ID NO: 2) (Taplin et al., Cancer Res., 59: 2511-2515, 1999). On this type of mutant ARs, some antiandrogen drugs, including flutamide, conversely exhibited an action to stimulate cancer cell proliferation (Veldscholte et al., Biochem. Biophys. Res. Commun., 173: 534-540, 1990). Also, although missense mutations of AR were identified in 3 of 11 biopsy samples from patients who experienced relapsed prostate cancer after an endocrine therapy with a combination of bicalutamide and surgical castration, all mutation sites were other than the 877th amino acid, which is a prevalent mutation site in flutamide-resistant relapsed cancers (Haapala et al., Lab. Invest., 81: 1647-1651, 2001).

In developing a drug useful for the prophylaxis/treatment of androgen-independent cancers based on AR mutations, cancer cell lines that express such mutant ARs are very useful research tools. However, all mutant-AR-expressing cancer cells that have been reported to date were clinically obtained from relapsed cancers or pre-treatment prostate cancer; there is no case wherein a mutation was induced in AR-expressing cancer cells *in vitro* to establish a cancer cell line that expresses an AR having a mutation similar to those of clinically found mutant ARs derived from androgen-independent cancers. Also, mutant ARs clinically found in androgen-independent cancers mostly have a single mutation at the amino acid level; reported cases of ARs having amino acid mutations at 2 sites or more are not unavailable but are very rare.

Accordingly, the object of the present invention is to provide a method of producing in *vitro* a cancer cell line that expresses an AR having a mutation similar to those of clinically found mutated ARs derived from androgen-independent cancers, and the mutant AR and the cancer cell line that expresses it which are obtained by said method, and to provide a method of screening a drug effective in the prophylaxis/treatment of androgen-independent relapsed cancers to endocrine therapy using them.

Disclosure of the Invention

The present inventors conducted extensive investigations with the aim of accomplishing the above-described object, and found that when human prostate cells that are sensitive to a specified antiandrogen drug are cultured in the presence of said antiandrogen drug for a long period, a mutation to cause the antiandrogen drug to considerably lose potency or conversely exhibit agonist-like action is induced in an AR, and, as a result, the cells become an antiandrogen-drug-resistant, that is, androgen-independent, cancer and begin proliferation. Furthermore, the present inventors isolated the cDNA of the AR from cancer cells showing proliferation, analyzed the entire base sequence thereof, and found that the mutation site of the AR agrees well with that of a mutant AR clinically obtained from a relapsed cancer in a patient undergoing an endocrine therapy with an antiandrogen drug. The present inventors conducted further investigations based on these findings, which resulted in the completion of the present invention.

Brief Description of the Drawings

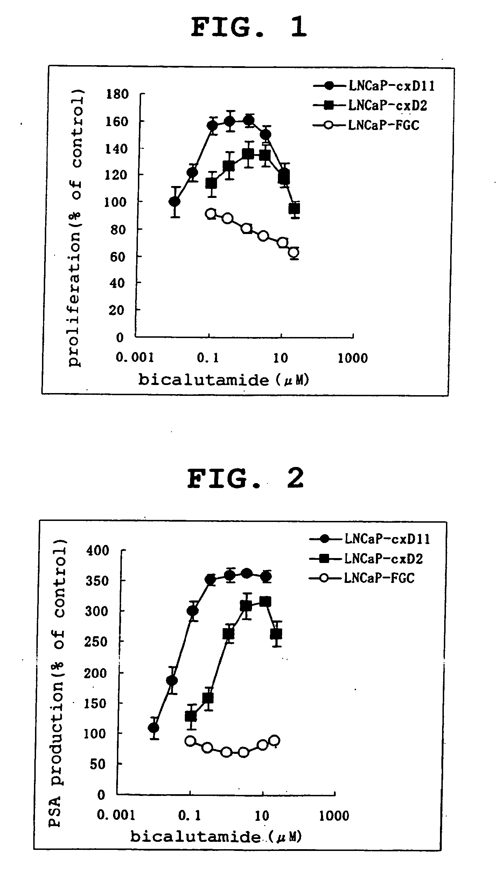

Figure 1 is a drawing showing the effects of bicalutamide on the proliferation of LNCaP-cxD11, LNCaP-cxD2 and LNCaP-FGC cells. LNCaP-caD11, LNCaP-cxD2 and LNCaP-FGC cells suspended in a medium (RPM11640 +10% DCC-FBS) were sown to 24-well plates at 40000 cells/plate, and, on the following day, bicalutamide was added. Three days after bicalutamide addition, cells were counted. Mean $\pm$ standard error, n=3.

Figure 2 is a drawing showing the effects of bicalutamide on the PSA production by LNCaP-caD11, LNCaP-cxD2 and LNCaP-FGC cells. LNCaP-cxD11, LNCaP-cxD2 and LNCaP-FGC cells suspended in a medium (RPM11640 + 10% DCC-FBS) were sown to 24-well plates at 40000 cells/plate, and, on the following day, bicalutamide was added. Three days after bicalutamide addition, supernatant concentrations of PSA were measured. Mean $\pm$ standard error, n=3.

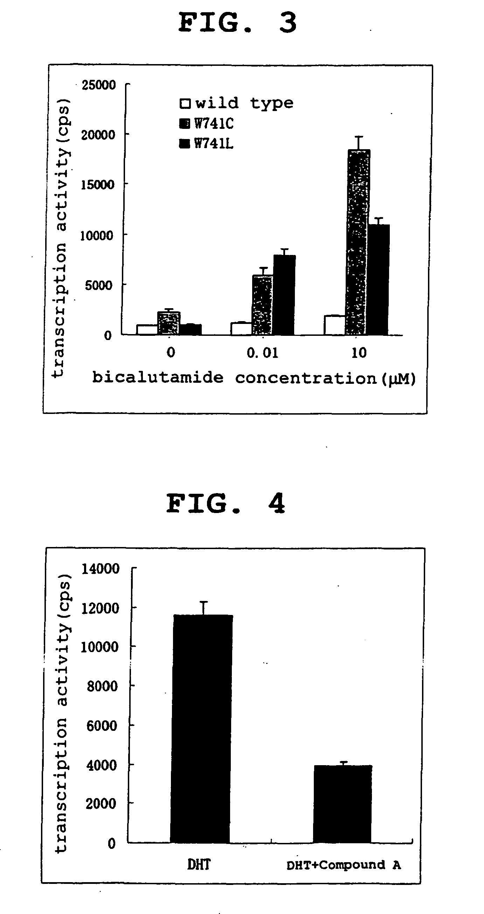

Figure 3 is a drawing showing the transcription-promoting activity of bicalutamide on wild type or mutant (W741C, W741L) ARs. A vector DNA incorporating each AR and a vector DNA having the luciferase gene linked downstream of an androgen-responsive promoter were co-transfected, and 0.01 or 1 $\mu$M bicalutamide was added, after which luciferase activity was measured. Mean $\pm$ standard error, n=4.

Figure 4 is a drawing showing the antagonistic action of Compound A on a W741C type mutant AR. A vector DNA incorporating the W741C type mutant AR and a vector DNA having the luciferase gene linked downstream of an androgen-responsive promoter were co-transfected, and 40nM DHT or both 40nM DHT and 10 $\mu$M Compound A were added, after which luciferase activity was measured. Mean $\pm$ standard error, n=16 for the only-DHT addition group and n=4 for the DHT+Compound A addition group.

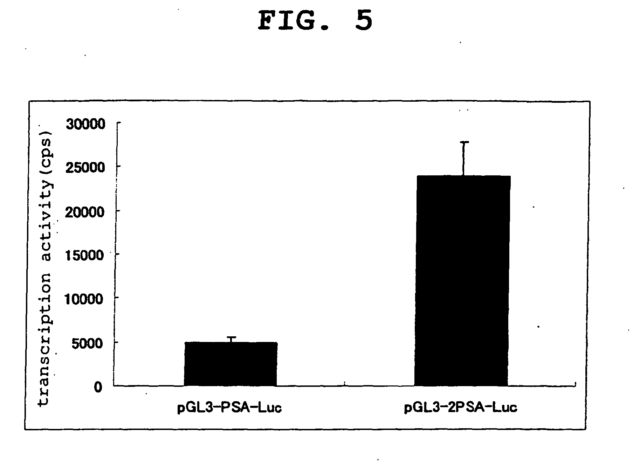

Figure 5 is a drawing showing the transcription activation of a gene under the control of the PSA promoter by a wild type AR activated by DHT. A vector DNA incorporating the wild type AR and a vector DNA having the luciferase gene linked downstream of one PSA promoter or two tandemly ligated PSA promoters were co-transfected to COS-7 cells, and 1 $\mu$M DHT was added, after which luciferase activity was measured. Mean $\pm$ standard error, n=6.

The mutant AR of the present invention is a protein containing the amino acid sequence in which tryptophan at amino acid number 746 is substituted by leucine in the amino acid sequence represented by SEQ ID NO: 2 (Proc. Natl. Acad. Sci. USA, 85: 7211-7215, 1988) (also abbreviated as "W746L"), or an amino acid sequence in which tryptophan at amino acid number 746 is substituted by leucine or cysteine and threonine at amino acid number 882 is substituted by alanine in the amino acid sequence represented by SEQ ID NO: 2 (also abbreviated as "W746L(C)+T882A").
The mutant AR of the present invention may be any protein derived from any cells (e.g., splenocytes, nerve cells, glial cells, beta cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, pituitary, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testicle, testis, ovary, placenta, uterus, bone, joint, skeletal muscle, etc. (particularly prostate) from mammals (e.g., human, guinea pig, rats, mouse, rabbit, swine, sheep, bovine, monkey, etc.). The mutant AR may also be a synthetic protein.

Throughout the present specification, proteins are represented in accordance with the conventional way of describing peptides, that is, the N-terminus (amino terminus) at the left hand side and the C-terminus (carboxyl terminus) at the right hand side. In the mutant AR of the present invention, the C-terminus may be usually in the form of a carboxyl group ($-COOH$), a carboxylate ($-COO^-$), an amide ($-CONH_2$) or an ester ($-COOR$).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, alpha-naphthyl, etc.; a $C_{7-14}$ aralkyl group such as a phenyl-$C_{1-2}$-alkyl group, e.g., benzyl, phenethyl, etc., or an $\alpha$-naphthyl-$C_{1-2}$-alkyl group such as $\alpha$-naphthylmethyl, etc.; and the like. In addition, pivaloyloxymethyl or the like, which is used widely as an ester for oral administration, may also be used.

When the mutant AR of the present invention has a carboxyl group (or a carboxylate) at a position other than the C-terminus, it may be amidated or esterified and such an amide or ester is also included within the mutant AR of the present invention. In this case, as examples of the ester, the above-described ester at the C-terminus and the like can be used.

Furthermore, examples of the mutant AR of the present invention include, referring to the above-described protein, those wherein the amino group of the methionine residue at the N-terminus is protected by a protecting group (for example, a $C_{1-6}$ acyl group such as a $C_{2-6}$ alkanoyl group such as a formyl group or acetyl, and the like), those wherein the glutamyl group resulting from cleavage on the N-terminus side *in vivo* is pyroglutamated, those wherein a substituent on the side chain of an amino acid in the molecule (for example, -OH, -SH, amino group, imidazole group, indole group, guanidino group and the like) is protected by an appropriate protecting group (for example, a $C_{1-6}$ acyl group such as a $C_{2-6}$ alkanoyl group such as a formyl group or acetyl, and the like); or complex proteins such as what is called a glycoprotein, wherein a sugar chain is bound, and the like.

Specific examples of the mutant AR of the present invention include a human-derived (more preferably human prostate cancer-derived) AR containing W746L or W746L+T882A.

For salts of the mutant AR of the present invention, preferred are salts with physiologically acceptable acids or bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The mutant AR of the present invention or a salt thereof may be produced by isolating and purifying from animal cells having an ability of producing said mutant AR using a publicly known method for purifying a receptor protein. For example, it can be purified and isolated by homogenizing human or mammalian tissues followed by extraction with an acid or the like, and subjecting the extract to a combination of chromatography techniques such as reverse phase chromatography, ion exchange chromatography and the like.

Examples of animal cells having an ability of producing the mutant AR of the present invention include the aforementioned human or mammalian cells or tissues (particularly cells derived from a relapsed cancer of a prostate cancer patient receiving an antiandrogen drug (e.g., bicalutamide, or bicalutamide and flutamide)), an antiandrogen drug-resistant cancer cell line obtained by culturing a cancer cell line that expresses a normal AR or an AR having the T882A mutation (e.g., LNCaP cell line) in the presence of an antiandrogen drug (e.g., bicalutamide or an analogue thereof) followed by selection using cell proliferation as an index, and the like.

The mutant AR of the present invention or a salt thereof may also be produced by culturing a transformant transformed with a recombinant vector that contains a DNA encoding the mutant AR of the present invention described below. Alternatively, it may also be produced by the protein synthesis methods to be hereinafter described or modifications thereof, based on the amino acid sequence of W746L or W746L+T882A.

To synthesize the mutant AR of the present invention, or salts or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the receptor protein is cut out from the resin and at the same time, the protecting groups are removed. Then, intramolecular disulfide bond-forming reaction is performed in a highly diluted solution to obtain the objective protein or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N, N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, tertiary butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. Both proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The mutant AR of the present invention can be produced by publicly known methods for peptide synthesis. For the methods for peptide synthesis, for example, either solid phase synthesis or liquid phase synthesis may be used. That is, the partial peptide or amino acids that can construct the mutant AR of the present invention or the partial peptide of the present invention are condensed with the remaining part. Where the product contains protecting groups, these protecting groups are removed to give the desired peptide. Publicly known methods for condensation and elimination of the protecting groups are described in (1)-(5) below:

(1) M. Bodanszky & M.A. Ondetti: Peptide Synthesis, Interscience Publishers, New York (1966);

(2) Schroeder & Luebke: The Peptide, Academic Press, New York (1965);

(3) Nobuo Izumiya, et al.: Peptide Gosei-no-Kiso to Jikken (Basics and experiments of peptide synthesis), published by Maruzen Co. (1975);

(4) Haruaki Yajima & Shunpei Sakakibara: Seikagaku Jikken Koza (Biochemical Experiment) 1, Tanpakushitsu no Kagaku (Chemistry of Proteins) IV, 205 (1977);

(5) Haruaki Yajima, ed.: Zoku Iyakuhin no Kaihatsu (A sequel to Development of Pharmaceuticals), Vol. 14, Peptide Synthesis, published by Hirokawa Shoten.

Also, after completion of the reaction, the mutant AR of the present invention may be purified and isolated using a combination of conventional purification methods such as solvent extraction, distillation, column chromatography, liquid chromatography, recrystallization and the like. When the mutant AR of the present invention obtained by the above methods is in a free form, it can be converted into an appropriate salt by a publicly known method; when it is obtained in a salt form, it can be converted into a free form by a publicly known method.

The polynucleotide encoding the mutant AR of the present invention may be any polynucleotide so long as it contains the base sequence (DNA or RNA, preferably DNA) encoding the mutant AR of the present invention described above. Such a polynucleotide may also be any one of DNA encoding the mutant AR of the present invention, RNA such as mRNA, etc., and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the mutant AR of the present invention, mRNA of the mutant AR of the present invention can be quantified by, for example, the publicly known method published in separate volume of Jikken Igaku 15 (7) "New PCR and its application" (1997) and the like, or by its modifications.

The DNA encoding the mutant AR of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the mutant AR of the present invention may be any DNA, as long as it is a DNA having a base sequence in which the base at base number 2237 is substituted by thymine in the base sequence represented by SEQ ID NO: 1 (G2237T) or a base sequence in which the base at base number 2237 is substituted by thymine and the base at base number 2644 is substituted by guanine in the base sequence represented by SEQ ID NO: 1 (G2237 T+A2644G).

For cloning of the DNA that completely encodes the mutant AR of the present invention, the DNA may be either amplified by PCR method using synthetic DNA primers containing a part of the base sequence encoding the mutant AR of the present invention, or selected by hybridizing the DNA inserted into an appropriate vector with a labeled DNA fragment or synthetic DNA that encodes a part or entire region of the mutant AR of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the Gapped duplex method or the Kunkel method or its modification by using a publicly known kit available as Mutan™-G or Mutan™-K (both manufactured by Takara Shuzo Co., Ltd.).

The cloned DNA encoding the mutant AR can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the mutant AR of the present invention can be manufactured, for example, by (a) excising the desired DNA fragment from the DNA encoding the mutant AR of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SR α promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, $\lambda P_L$ promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHOS promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp[r]), neomycin resistant gene (hereinafter sometimes abbreviated as Neo[r], G418 resistance), etc. In particular, when dhfr gene is used as the selection marker in CHO (dhfr[-]) cells, selection can also be made on thymidine free media.

If necessary, a signal sequence that matches with a host is added to the N-terminus of the AR of the present invention. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the mutant AR of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R[-], NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of silkworm can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr[-]) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector containing the DNA encoding the AR can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogen phosphate, magnesium chloride, etc. In addition, yeast extract, vitamins, growth promoting factors etc. may also be added to the medium. Preferably, pH of the medium is adjusted to about 5 to about 8.

A preferred example of the medium for cultivation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary, the culture may be aerated or agitated.

Where the bacteria belonging to the genus Bacillus are used as the host, the transformant is cultivated generally at about 30°C to about 40°C for about 6 hours to about 24 hours. If necessary, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the culture is performed at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The culture is usually done at about 30°C to about 40°C for about 15 hours to about 60 hours and, if necessary, the culture can be aerated or agitated.

As described above, the mutant AR of the present invention can be produced within the cell or out of the cell of the transformant.

The mutant AR of the present invention can be separated and purified from the culture described above by the following procedures.

When the mutant AR of the present invention is extracted from the cultured bacteria or cells, after cultivation these cells are collected by a publicly known method and suspended in a appropriate buffer. The bacteria or cells are then disrupted by publicly known methods such as ultrasonication, a treatment with lysozyme and/or freeze-thaw cycling, followed by centrifugation, filtration, etc. Thus, the crude extract of the mutant AR of the present invention can be obtained. The buffer used for the procedures may contain a protein modifier such as urea or guanidine hydrochloride, or a surfactant such as Triton X-100™, etc. When the mutant AR of the present invention is secreted in the culture, after completion of the cultivation the supernatant can be separated from the bacteria or cells to collect the supernatant by a publicly known method.

The AR of the present invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the mutant AR of the present invention thus obtained is in a free form, it can be converted into the salt form by publicly known methods or modifications thereof. On the other hand, when the mutant AR is obtained in the salt form, it can be converted into the free form or in the form of a different salt by publicly known methods or modifications thereof.

The mutant AR of the present invention produced by the recombinant can be treated, prior to or after the purification, with an appropriate protein modifying enzyme so that the mutant AR can be appropriately modified and partially removed a polypeptide. Examples of the protein-modifying enzyme include trypsin, chymotrypsin, arginyl endopeptidase, protein kinase, glycosidase or the like.

The activity of the thus produced mutant AR of the present invention or salts thereof can be determined by a binding experiment to a labeled ligand, by an enzyme immunoassay using the antibody of the present invention (described in detail below) and the like.

A DNA that encodes the mutant AR of the present invention or a salt thereof, or an amide thereof or an ester thereof or a salt thereof can be used for (1) an agent for the prophylaxis and/or treatment of diseases that can be ameliorated by overexpression of the mutant AR of the present invention, (2) a genetic diagnostic agent, (3) a method of screening for a compound that alters the expression level of the mutant AR of the present invention or a salt thereof, (4) an agent for the prophylaxis and/or treatment of various diseases, which comprises a compound that alters the expression level of the mutant AR of the present invention, (5) a method of screening for a compound that alters the bindability of the mutant AR of the present invention and ligand (agonist, antagonist and the like), (6) an agent for the prophylaxis and/or treatment of various diseases, which comprises a compound that alters the bindability of the mutant AR of the present invention and ligand (agonist, antagonist), (7) quantitation of the mutant AR of the present invention, (8) a pharmaceutical agent comprising the antibody of the present invention, (9) a pharmaceutical agent comprising an antisense DNA, (10) production of a DNA-introduced animal, and the like.

Particularly, by a screening with a receptor binding assay system using an animal cell capable of expressing the mutant AR of the present invention or an expression system of a recombinant mutant AR, a compound that alters the bindability of a ligand for a human- or mammal-specific receptor (e.g., agonist, antagonist and the like) can be selected, and said agonist or antagonist can be used as an agent for the prophylaxis/treatment of various diseases, and the like.

The intended uses of the mutant AR of the present invention, the DNA that encodes the mutant AR of the present invention (hereinafter also abbreviated as the DNA of the present invention), are specifically described below.

(1) Prophylactic and/or therapeutic agent for diseases that can be ameliorated by overexpression of the mutant AR of the present invention

The mutant AR of the present invention is **characterized in that** bicalutamide or an analogue thereof, or further flutamide or an analogue thereof, acts thereon as an agonist. Because it is known that in prostate cancer, a stimulation of AR in excess conversely suppresses proliferation, by locally delivering (1) the mutant AR of the present invention or (2) the DNA of the present invention to cancer cells and allowing it in excess therein, it is possible to suppress the proliferation of antiandrogen drug-resistant cancer cells.

When the mutant AR of the present invention is used as the above-described prophylactic/therapeutic agent, it can be made into a preparation in a conventional manner.

On the other hand, where the DNA of the present invention is used as the above-described prophylactic/ therapeutic agent, the DNA of the present invention itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, (1) the mutant AR of the present invention or b) the DNA of the present invention can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. For example, these preparations can be manufactured by mixing (1) the mutant AR of the present invention or (2) the DNA of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained preparation is safe and low toxic, it can be administered to mammals (e.g., human, rat, rabbit, sheep, swine, bovine, cat, dog, monkey and the like).

The dose of the mutant AR of the present invention varies depending on subject to be administered, organs to be administered, conditions, routes for administration, etc.; in oral administration, e.g., for the patient, with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(2) Genetic diagnostic reagent

By using the DNA of the present invention as a probe, the DNA or mRNA encoding the mutant AR of the present invention (i.e., an abnormality in a normal AR gene or mRNA) in mammals (e.g., human, rat, rabbit, sheep, swine, bovine, cat, dog, monkey and the like) can be detected. Therefore, it is useful as a genetic diagnostic agent for a cancer, particularly as a genetic diagnostic agent for the development, increase and the like of a cancer cell that has acquired resistance to an antiandrogen drug (e.g., bicalutamide, flutamide and the like).

The genetic diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

(3) Method of screening for a compound that alters the expression level of the mutant AR of the present invention

By using as a probe, the DNA of the present invention can be used for screening for a compound that alters the expression level of the mutant AR of the present invention.

That is, the present invention provides a method of screening for a compound that alters the expression level of the mutant AR of the present invention, which comprises measuring the amount of mRNA encoding the mutant AR of the present invention contained in, for example, (i) (1) blood, (2) specific organs, (3) tissues or cells isolated from the organs of non-human mammals (e.g., rat, rabbit, sheep, swine, bovine, cat, dog, monkey and the like), or in (ii) transformants, etc.

The amount of mRNA encoding mutant AR of the present invention can be specifically measured as follows.

(i) A non-human mammal (e.g., mouse, rat, rabbit, sheep, swine, bovine, cat, dog, monkey and the like) having cells that express a normal AR or the mutant AR of the present invention receive administration of a drug or physical stress, and blood, specific organs, or tissues or cells isolated from the organs are obtained after a specified period of time. The mRNA encoding the mutant AR of the present invention contained in the thus obtained cells is extracted from the cells, for example, in a conventional manner and quantified using, e.g., TaqManPCR, or may also be analyzed by Northern blot technique by publicly known methods.

(ii) A Transformant that expresses the mutant AR of the present invention or an animal cell capable of expressing the mutant AR of the present invention is prepared according to the methods described above, and the mRNA encoding the AR of the present invention can be quantified and analyzed in the same manner.

Screening for a compound that alters the expression level of the mutant AR of the present invention can be coducted by the following procedures:

(i) To a non-human mammal having cells that express a normal AR or the mutant AR of the present invention, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA encoding the mutant AR of the present invention contained in the cells is quantified and analyzed; or

(ii) A test compound is mixed in a culture medium and a transformant or an animal cell capable of expressing the mutant AR of the present invention is cultured therein in a conventional manner. After a specified time (after 1 day to 7 days, preferably after 1 day to 3 days, more preferably after 2 to 3 days), the amount of mRNA encoding the mutant AR of the present invention contained in the transformant or animal cell capable of expressing the mutant AR of the present invention can be quantified and analyzed.

The compound or its salt, which is obtainable by the screening method of the present invention, is a compound that alters the expression level of the mutant AR of the present invention, specifically, (a) a compound that excessively enhances a cell-stimulating activity (e.g., cell proliferation stimulation, PSA expression induction activity and the like) mediated by the mutant AR of the present invention by increasing the expression level of the mutant AR of the present invention, or (b) a compound that attenuates said cell-stimulating activity by decreasing the expression level of the mutant AR of the present invention.

The compound includes a peptide, protein, non-peptide compound, synthetic compound, and fermentation product. It may be a novel or known compound.

The compound that excessively enhances the cell-stimulating activity is capable of suppressing the proliferation of antiandrogen drug-resistant cancers by, for example, using it in combination with an antiandrogen drug, and is useful as a prophylactic/therapeutic agent for hormone-sensitive cancers (e.g., prostate cancer and the like) in the androgen-independent stage.

Since the compound that attenuates the cell-stimulating activity decreases the physiological activity of the mutant AR of the present invention, it is useful as a prophylactic/therapeutic agent for hormone-sensitive cancers (e.g., prostate cancer and the like) in the androgen-independent stage.

When the compound or its salt obtained by the screening method of the present invention is used as a pharmaceutical composition, the compound can be prepared into a pharmaceutical composition in a conventional manner. For example, in the same manner as the above-described pharmaceutical agent containing the mutant AR of the present invention, the compound can be made into tablets, capsules, elixirs, microcapsules, sterile solutions, suspensions and the like.

Since the thus obtained preparation is safe and low toxic, it can be administered to mammals (e.g., human, rat, rabbit, sheep, swine, bovine, cat, dog, monkey, etc.).

The dose of the compound or its salt varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(4) Prophylactic and/or therapeutic agent for various diseases, which comprises a compound that alters the expression level of the mutant AR of the present invention

A compound that alters the expression level of the mutant AR of the present invention can be used as a prophylactic/therapeutic agent for hormone-dependent and hormone-independent cancer (e.g., prostate cancer and the like). Said compound can be made into a preparation in a conventional manner.

For example, said compound can be used orally in the form of tablets which may be sugar coated if necessary, capsules, elixirs, microcapsules etc., or parenterally in the form of injectable preparations such as a sterile solution and a suspension in water or with other pharmaceutically acceptable liquid. For example, these preparations can be manufactured by mixing said compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained preparation is safe and low toxic, it can be administered to mammals (e.g., human, rat, rabbit, sheep, swine, bovine, cat, dog, monkey and the like).

The dose of the compound or salt thereof varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(5) Method of screening for a compound (agonist, antagonist and the like) that alters the bindability of the mutant AR of the present invention and a ligand

By using the mutant AR of the present invention, or by constructing an expression system for the recombinant mutant AR and using a receptor binding assay system with the above expression system, a compound that alters the bindability of a ligand and the mutant AR of the present invention (e.g., peptide, protein, non-peptide compound, synthetic compound, fermentation product, etc.), or a salt thereof, can be screened efficiently.

Such compounds include (a) a compound that has an activity to promote a cell-stimulating activity (e.g., cell proliferation activity, PSA expression induction activity and the like) via the mutant AR of the present invention (so-called agonist for the mutant AR of the present invention), (b) a compound that has no cell-stimulating activity (so-called antagonist for the mutant AR of the present invention), (c) a compound that enhances the binding force of a ligand and the mutant AR of the present invention, or (d) a compound that decreases the binding force of a ligand and the mutant AR of the present invention and the like.

That is, the present invention provides a method of screening for a compound that alters the bindability of a ligand and the mutant AR of the present invention or a salt thereof, which comprises comparing (i) the case wherein the mutant AR of the present invention is brought into contact with a ligand, with (ii) the case wherein the mutant AR of the present invention is brought into contact with a ligand and a test compound.

The screening method of the present invention is characterized by comprising determining, for example, the amount bound of ligand (androgen) for the AR of the present invention, a cell-stimulating activity (e.g., cell proliferation activity, PSA expression induction activity) and the like, in cases (i) and (ii), and comparing both cases.

More specifically, the present invention provides:

(1) a method of screening for a compound that alters the bindability of a ligand and the mutant AR of the present invention, or a salt thereof, which comprises measuring the amounts of a labeled ligand bound to the mutant AR of the present invention, when the labeled ligand is brought into contact with said mutant AR and when the labeled ligand and a test compound are brought into contact with said mutant AR, and comparing the both amounts,

(2) a method of screening for a compound that alters the bindability of a ligand and the mutant AR of the present invention, or a salt thereof, which comprises measuring the amounts of labeled ligand bound to cells containing the mutant AR of the present invention (animal cells capable of expressing the mutant AR, and the like), when the labeled ligand is brought into contact with said cells and when the labeled ligand and a test compound are brought into contact with said cells, and comparing the both amounts,

(3) a method of screening for a compound that alters the bindability of a ligand and the mutant AR of the present invention, or a salt thereof, which comprises measuring the amounts of labeled ligand bound to the mutant AR, when the labeled ligand is brought into contact with the mutant AR expressed by culturing a transformant containing the DNA of the present invention and when the labeled ligand and a test compound are brought into contact with the mutant AR of the present invention expressed by culturing a transformant containing the DNA of the present invention, and comparing the both amounts, and

(4) a method of screening for a compound that alters the bindability of a ligand and the mutant AR of the present invention, or a salt thereof, which comprises measuring receptor-mediated cell-stimulating activities (e.g., cell proliferation activity, PSA expression induction activity and the like), when a compound that activates the mutant AR of the present invention (e.g., an androgen or an antiandrogen drug such as bicalutamide or flutamide, and the like) is brought into contact with cells containing the mutant AR of the present invention (animal cells capable of expressing the mutant AR) and when the compound that activates the mutant AR of the present invention and a test compound are brought into contact with said cells, and comparing the both activities.

Hereinafter, the screening method of the present invention is described specifically.

First, for the mutant AR of the present invention used for the screening method of the present invention, any one may be used so long as it contains the mutant AR of the present invention described above. The aforementioned animal cells capable of expressing the mutant AR of the present invention, and the like are preferred.

To manufacture the mutant AR of the present invention, the methods described above are used, and it is preferred to express the DNA of the present invention in mammalian and insect cells. For the DNA fragment encoding the objective protein region, the complementary DNA, but not necessarily limited thereto, is employed. For example, the gene fragments and synthetic DNA may also be used. To introduce a DNA fragment encoding the mutant AR of the present invention into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SRa promoter. The amount and quality of the expressed receptor are examined by publicly known methods, for example, the method described in the literature [Nambi, P. et al., The Journal of Biological Chemistry (J. Biol. Chem.), 267, 19555-19559, 1992].

Therefore, in the screening method of the present invention, as a material that contains the mutant AR of the present invention, the mutant AR purified by publicly known methods or cells containing the mutant AR may be used.

In the screening method of the present invention, when cells containing the mutant AR of the present invention are used, the cells may be fixed with glutaraldehyde, formalin, etc. The cells can be fixed by publicly known methods.

The cells containing the mutant AR of the present invention are host cells that express said mutant AR. For the host cells, *Escherichia coli, Bacillus subtilis,* yeast, insect cells, animal cells and the like are preferred.

The amount of the mutant AR in the cells containing the mutant AR is preferably $10^3$ to $10^8$ molecules per cell, more preferably $10^5$ to $10^7$ molecules per cell. As the amount of expression increases, the ligand binding activity (specific activity) increases so that not only the highly sensitive screening system can be constructed but also large quantities of samples can be assayed with the same lot.

To perform the above-mentioned (1) to (3) to screen for a compound that alters the bindability of a ligand and the mutant AR of the present invention, for example, an appropriate mutant AR fraction and a labeled ligand are necessary.

The mutant AR fraction is preferably an AR fraction derived from cells expressing the mutant AR, which is obtained by a spontaneous or artificial mutation, or a recombinant mutant AR fraction having an activity equivalent thereto. Herein, equivalent activity is intended to mean an equivalent ligand binding activity, cell-stimulating activity or the like.

For the labeled ligand, a labeled ligand (androgen) and a labeled ligand analogue are used. For example, testosterone, DHT and the like labeled with [3H], [125I], [14C], [35S], etc. are used.

Specifically, to screen a compound that alters the bindabiliy of a ligand and the mutant AR of the present invention, first, the mutant AR standard is prepared by suspending cells containing the mutant AR of the present invention in a buffer appropriate for the screening. For the buffer, any buffer that does not interfere with the binding of the ligand to the mutant AR is usable and examples of such a buffer are phosphate buffer, Tris-hydrochloride buffer, etc., having pH of 4 to 10 (preferably pH of 6 to 8). To minimize a non-specific binding, a surfactant such as CHAPS, Tween-80™ (Kao-Atlas Co.), digitonin, deoxycholate, etc. may be added to the buffer. To inhibit degradation of the receptor and ligands by proteases, protease inhibitors such as PMSF, leupeptin, E-64 (manufactured by Peptide Research Laboratory, Co.), and pepstatin may be added. To 0.01 to 10 ml of the receptor solution, a given amount (5,000 to 500,000 cpm) of labeled ligand is added, and $10^{-4}$ M - $10^{-10}$ M of a test compound is simultaneously added to be co-present. To examine non-specific binding (NSB), a reaction tube containing an unlabeled test compound in large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. Regarding the count obtained by subtracting the amount of non-specific binding (NSB) from the count obtained in the absence of any competitive substance ($B_0$) as 100%, when the amount of specific binding (B-NSB) is, for example, 50% or less, the test compound can be selected as a candidate substance having a potential of competitive inhibition.

Of the method of (4) above to screen for a compound that alters the bindability of a ligand and the mutant AR of the present invention, the most preferable mode is described in detail below.

Generally, the cells containing the mutant AR of the present invention are first cultured on a multi-well plate, etc. Prior to screening, the medium is replaced with fresh medium or with an appropriate non-cytotoxic buffer, followed by incubation for a given period of time in the presence of a test compound, etc. Subsequently, the extent of proliferation of the cells is evaluated, or the cells are extracted or the supernatant is recovered and the resulting product is quantified by appropriate procedures. Where it is difficult to detect the production of the index substance (e.g., PSA and the like) for the cell-stimulating activity due to a degrading enzyme contained in the cells, an inhibitor against such a degrading enzyme may be added prior to the assay.

Screening by assaying the cell-stimulating activity requires cells that have expressed an appropriate mutant AR. For the cells that have expressed the mutant AR of the present invention, cells expressing the mutant AR, which is obtained by a spontaneous or artificial mutation, the cell line expressing the recombinant mutant AR described above and the like are desirable.

The test compound includes peptide, protein, non-peptide compound, synthetic compound, fermentation product, cell extract, plant extract, animal tissue extract and the like. These compounds may be new compounds or known compounds.

The kit for screening for a compound that alters the bindability of a ligand and the mutant AR of the present invention is a kit, which comprises the mutant AR of the present invention or cells containing the mutant AR of the present invention (animal cells capable of expressing the mutant AR and the like), and the like.

Examples of the screening kit of the present invention are as follow.

1. Reagents for screening

(1) Buffer for measurement and washing

Hanks' balanced salt solution (Gibco) supplemented with 0.05% bovine serum albumin (Sigma).
The solution is sterilized by filtration through a 0.45 μm filter, and stored at 4°C or may be prepared at use.

(2) Standard mutant AR

CHO cells expressing the mutant AR of the present invention are passaged in a 12-well plate at a density of $5 \times 10^5$ cells/well followed by culturing at 37°C under 5% $CO_2$ and 95% air for 2 days.

(3) Labeled ligands

A solution of DHT labeled with commercially available [$^3$H], [$^{125}$I], [$^{14}$C], [$^{35}$S], etc. is stored at 4°C or -20°C, and diluted to 1 μM with the measurement buffer.

(4) Standard ligand solution

DHT is dissolved in and adjusted to 1 mM with PBS containing 0.1% bovine serum albumin (Sigma) and stored at -20°C.

2. Measurement method

(1) CHO cells expressing the mutant AR of the present invention are cultured in a 12-well culture plate and washed twice with 1 ml of the measurement buffer, and 490 $\mu$l of the measurement buffer is added to each well.
(2) After adding 5 $\mu$l of $10^{-3}$ - $10^{-10}$ M test compound solution, 5 $\mu$l of a labeled ligand is added to the mixture, and the cells are incubated at room temperature for an hour. To determine the amount of the non-specific binding, 5 $\mu$l of the non-labeled ligand is added in place of the test compound.
(3) The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)
(4) The radioactivity is measured using a liquid scintillation counter (manufactured by Beckman Co.), and the percent maximum binding (PMB) is calculated by the equation below [Equation 1].

$$[\text{Equation 1}]$$

$$PMB = [(B - NSB)/(B_0 - NSB)] \times 100$$

PMB: Percent maximum binding
B: Value obtained in the presence of a test compound
NSB: Non-specific binding
Bo: Maximum binding

The compound or its salt, which is obtained using the screening method or the screening kit of the present invention, is a compound that alters the bindability of a ligand and the mutant AR of the present invention. Specifically, it is: (a) a compound that has the mutant AR-mediated cell-stimulating activity (e.g., cell proliferation activity, PSA production, etc.) (so-called agonist for the mutant AR of the present invention); (b) a compound having no cell stimulating-activity (so-called antagonist for the mutant AR of the present invention); (c) a compound that enhances the binding force between the ligand and the mutant AR of the present invention; or (d) a compound that attenuates the binding force between the ligand and the mutant AR of the present invention.

The compound includes a peptide, protein, non-peptide compound, synthetic compound, and fermentation product. It may be a novel or known compound.

Since antagonists to the mutant AR of the present invention can suppress the physiological activities of ligands (androgens) and agonists (some antiandrogen drugs such as bicalutamide and flutamide, and the like) for the mutant AR of the present invention, they are useful as a prophylactic/therapeutic agent for androgen-independent (antiandrogen drug-resistant) cancers, particularly prostate cancer.

A compound that attenuates the binding force between the ligand and the mutant AR of the present invention is useful as a safe and low toxic pharmaceutical agent (e.g., a prophylactic/therapeutic agent for androgen-independent (antiandrogen drug-resistant) cancers, particularly prostate cancer) for decreasing the physiological activities of a ligand (agonist) for the mutant AR of the present invention.

When the compound or salt thereof obtained by using the screening method or the screening kit of the present invention, is used as the aforementioned pharmaceutical composition, the pharmaceutical preparation can be obtained in a conventional manner. For example, in the same manner as the above-described pharmaceutical agent comprising the mutant AR of the present invention, the compound can be made into tablets, capsules, elixirs, microcapsules, sterile solutions, suspensions and the like.

Since the thus obtained preparation is safe and low toxic, it can be administered to mammals (e.g., human, rat, rabbit, sheep, swine, bovine, cat, dog, monkey and the like).

The dose of the compound or salt thereof varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(6) Prophylactic and/or therapeutic agent for various diseases, which comprises a compound that alters the bindability of the mutant AR of the present invention and a ligand (agonist, antagonist)

Since antagonists for the mutant AR of the present invention can suppress the physiological activities for ligands (androgens) and agonists (some antiandrogen drugs such as bicalutamide and flutamide, and the like) for the mutant AR of the present invention, they can be used as a prophylactic/therapeutic agent for androgen-independent (antiandrogen drug-resistant) cancers, particularly prostate cancer. When said compound is used for the above-described diseases, it can be made into a preparation according to a conventional manner.

For example, said compound can be used orally in the form of tablets which may be sugar coated if necessary, capsules, elixirs, microcapsules etc., or parenterally in the form of injectable preparations such as a sterile solution and a suspension in water or with other pharmaceutically acceptable liquids. For example, these preparations can be manufactured by mixing said compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as, oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained preparation is safe and low toxic, it can be administered to mammals (e.g., human, rat, rabbit, sheep, swine, bovine, cat, dog, monkey and the like).

The dose of the compound or salt thereof varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

When bases, amino acids and the like are shown in abbreviations in the present specification and drawings, they are based on the abbreviations by the IUPAC-IUB Commission on Biochemical Nomenclature or abbreviations conventionally used in the pertinent field; examples thereof are given below. When an amino acid can have an optical isomer, it is the L-configuration unless otherwise specified.

DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
DTTP: Deoxythymidine triphosphate
dGTP: Deoxyguanosine triphosphate

dCTP: Deoxycytidine triphosphate
ATP: Adenosine triphosphate
EDTA: Ethylenediamine tetraacetate
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyroglutamic acid
Me: Methyl group
Et: Ethyl group
Bu: Butyl group
Ph: Phenyl group
TC: Thiazolidine-4 (R)-carboxamide group

Also, substituents, protecting groups and reagents often mentioned in the present specification are designated with the following symbols.

Tos: p-Toluenesulfonyl
CHO: Formyl
Bzl: Benzyl
Cl$_2$Bzl: 2,6-Dichlorobenzyl
Bom: Benzyloxymethyl
Z: Benzyloxycarbonyl
Cl-Z: 2-Chlorobenzyloxycarbonyl
Br-Z: 2-Bromobenzyloxycarbonyl
Boc: t-Butoxycarbonyl
DNP: Dinitrophenol
Trt: Trityl
Bum: t-Butoxymethyl
Fmoc: N-9-fluorenylmetboxycarbonyl
HOBt: 1-Hydroxybenztriazole
HOOBt: 3,4-Dihydro-3-hydroxy-4-oxo-1,2,3-benzotriazine
HONB: 1-Hydroxy-5-norbomane-2,3-dicarboxyimide
DCC: N, N'-dicyclohexylcarbodiimide

Sequence identification numbers in the sequence listing in the present specification indicate the following sequences.
[SEQ ID NO: 1]
Shows the base sequence of cDNA of wild type human AR.
[SEQ ID NO: 2]
Shows the amino acid sequence of the wild type human AR.

[SEQ ID NO: 3]
Shows an ARE sequence in the human PSA promoter.
[SEQ ID NO: 4]
Shows an ARR sequence in the human PSA promoter.
[SEQ ID NO: 5]
Shows an oligonucleotide designed to act as a primer for amplifying the human PSA promoter.
[SEQ ID NO: 6]
Shows an oligonucleotide designed to act as a primer for amplifying the human PSA promoter.

Free Texts in Sequence Listing

SEQ ID NO: 3: ARE sequence in human PSA promoter.
SEQ ID NO: 4: ARR sequence in human PSA promoter.
SEQ ID NO: 5: Oligonucleotide designed to act as primer for amplifying human PSA promoter.
SEQ ID NO: 6: Oligonucleotide designed to act as primer for amplifying human PSA promoter.

Example

The present invention is described in more detail by means of the following Examples, which are not to be construed as limiting the scope of the present invention.

Example 1: Method of establishing the LNCaP-cxD cell line

When LNCaP-FGC (ATCC Number: CRL-1740) is cultured in a culture broth (RPMI1640 + 10% Dextran Charcoal (DCC)-Fetal Bovine Serum (FBS)) containing 0.1 and 1 μM bicalutamide (commercial name: Casodex), it does not proliferate initially. However, when cultivation was continued for 6 weeks to 13 weeks or more, two cell lines exhibiting proliferation were obtained. These cells were designated LNCaP-cxD11 any LNCaP-cxD2, respectively.

Example 2: Bicalutamide response of the LNCaP-cxD cell line

LNCaP-cxD11, LNCaP-cxD2 and LNCaP-FGC were sown to 24-well plates at 40000 cells/mL/well, on the following day 0.1-30 μM bicalutamide was added, and 3 days after addition, cells were counted. Also, at this time, the concentration of androgen-dependently produced PSA (Prostatic Specific Antigen) in the culture supernatant was measured.
As a result, the proliferation of LNCaP-czD11 and LNCaP-cxD2 was significantly promoted by bicalutamide [Fig. 1]. On the other hand, in the parent line LNCaP-FGC, proliferation was significantly suppressed by bicalutamide [Fig. 1]. Also, PSA production was significantly promoted by bicalutamide in LNCaP-cxD11 and LNCaP-cxD2 and significantly suppressed by bicalutamide in the parent line [Fig. 2].

Example 3: Identification of mutant ARs

After total RNA was extracted from LNCaP-cxD11, LNCaP-cxD2 and LNCaP-FGC, it was converted to cDNA, and the base sequences of the AR genes were analyzed by the PCR direct sequencing method. For LNCaP-cxD11, only a portion, the androgen-binding region, of the AR gene was subjected to sequence analysis.
As a result, in both LNCaP-cxD11 and LNCaP-cxD2 ARs, one mutation in the gene sequence accompanied by an amino acid mutation was present in the androgen-binding region. The TGG (tryptophan) of codon 746 (normally a designation system to write the total number of codons of AR as 919, in which system this codon corresponds to codon 741) was found to be mutated to TTG (leucine) and TUT (cysteine) in LNCaP-cxD11 and LNCaP-cxD2, respectively.
Example 4: Transcription promoting activity of bicalutamide on mutated type AR 5,000,000 cells of Cos-7 were sown to a 150 cm$^2$ flask and cultured in a culture broth (DMEM + 10% Dextran Charcoal (DCC)-Fetal Bovine Serum (FBS) + 2mM glutamine) for 24 hours, after which a vector DNA incorporating a wild type AR or a mutated type AR (W741C and W741L type mutations) and a vector DNA having the luciferase gene bound downstream of an androgen-responsive promoter were co-transfected by the liposome method. Two hours later, the medium was replaced with a fresh one and 3 hours of cultivation was conducted, after which 0.01 or 1 μM bicalutamide was added and further 24 hours of cultivation was conducted, after which luciferase activity was measured and the AR transcription activity of bicalutamide was examined.
As a result, bicalutamide exhibited almost no transcription activity on the wild type, whereas bicalutamide exhibited transcription-promoting activity on the W741C and W741L type mutant ARs [Fig. 3].

Example 5: Screening of compound exhibiting antagonist action on W741C type mutant AR

A compound that inhibits the W741C type mutant AR transcription activity of DHT (dihydrotestosterone), namely a compound that exhibits antagonist action on the W741C type mutant AR was searched for by the same co-transfection system as the assay system described in Example 4; as a result, Compound A [ethyl 2,5-dimethyl-4-(4-nitrophenyl)-1H-pyrrole-3-carboxylate (manufactured by Labotest)] was found [Fig. 4].

Reference Example 1: Construction of expression vector containing a luciferase gene under the control of a PSA promoter

After genomic DNA was extracted from LNCaP-FGC cells according to a conventional method, a PCR reaction was conducted with this as the template using 5'-GGAGCTCGAATTCCACATTGTTTGCTGCACGTTGG-3' (SEQ ID NO: 5) and 5'-CAAGCTTTGGGGCTGGGGAGCCTCCCCCAGGAGC-3' (SEQ ID NO: 6) as the primers. Using Pyrobest (TaKaRa) as the DNA polymerase, the PCR reaction was conducted in 25 cycles of heating at 94°C for 1 minute, followed by heating at 98°C for 5 seconds, at 59°C for 30 seconds, and at 72°C for 1 minute, with the Gene Amp PCR System 9700 (Applied Biosystems), to yield an about 650 bp DNA fragment containing the PSA (prostate-specific antigen) promoter. After this fragment was cleaved with restriction endonucleases Hind **III** (TaKaRa) and Sac I (TaKaRa), it was subjected to agarose gel electrophoresis, and a DNA fragment was recovered. The DNA fragment was mixed with the pGL3-Basic vector (Promega), previously digested with Hind **III** and Sac I, and ligated using the Ligation high (TOYOBO), and *Escherichia coli* DH5α competent cells were transformed, to yield the vector pGL3-PSA-Luc, which has the luciferase gene ligated downstream of the PSA promoter. Furthermore, after pGL3-PSA-Luc was cleaved with Hind III, blunting was conducted using a Blunting kit (TaKaRa), after which the blunt-ended fragment was cleaved with Kpn I (TaKaRa) and a fragment containing the PSA promoter was recovered. After the DNA fragment was cleaved with Sac I, blunting was conducted using a Blunting kit, and the blunt-ended fragment was ligated to Kpn I-cleaved pGL3-PSA-Luc, and *Escherichia coli* DH5α competent cells were transformed, to yield pGL3-2PSA-Luc, which has two PSA promoters ligated tandem.

Example 6: Reporter assay system using a PSA promoter

5,000,000 cells of Cos-7 were sown to a 150 cm$^2$ flask and cultured in a culture broth (DMEM + 10% Dextran Charcoal (DCC)-Fetal Bovine Serum (FBS) + 2mM glutamine) for 24 hours, after which pGL3-PSA-Luc or pGL3-2PSA-Luc obtained in Reference Example 1 above and a vector DNA incorporating a wild type AR were co-transfected using SuperFect (Qiagen). Two hours later, the medium was replaced with a fresh one and 3 hours of cultivation was conducted, after which 1 μM DHT (5α-dihydrotestosterone) was added and further 24 hours of cultivation was conducted, after which luciferase activity was measured and transcription activity was examined. The results are shown in Fig. 5.

As is evident from Fig. 5, it was found possible to measure the effect of DHT on AR transcription activity using the PSA promoter. Furthermore, it was found possible to obtain more potent activity by tandemly linking the PSA promoter.

Industrial Applicability

By the method of the present invention, an effective prophylactic/therapeutic agent for androgen-independent relapsed prostate cancer can be screened. Also, development (relapse) of androgen-independent cancers can be detected early. Furthermore, by applying the new endocrine therapy introduced by the present invention, it is expected that the survival rate and period in prostate cancer is significantly improved.

**Claims**

1. A protein comprising the following amino acid sequence

   (a) or (b):
   (a) an amino acid sequence in which tryptophan at amino acid number 746 is substituted by leucine in the amino acid sequence represented by SEQ LID NO:2,
   (b) an amino acid sequence in which tryptophan at amino acid number 746 is substituted by leucine and threonine at amino acid number 882 is substituted by alanine in the amino acid sequence represented by SEQ ID NO:2, or a salt thereof.

2. A polynucleotide comprising a polynucleotide that encodes the protein of claim 1.

3. The polynucleotide of claim 2, which is a DNA.

4. The polynucleotide of claim 2, which has the following base sequence (a) or (b):

   (a) a base sequence in which the base at base number 2237 is substituted by thymine in the base sequence represented by SEQ ID NO:1,
   (b) a base sequence in which the base at base number 2237 is substituted by thymine and the base at base number 2644 is substituted by guanine in the base sequence represented by SEQ ID NO:1.

5. A diagnostic agent comprising the polynucleotide of claim 2.

6. The diagnostic agent of claim 5, which is for the diagnosis of hormone-independent cancers.

7. A recombinant vector comprising the polynucleotide of claim 2.

8. A non-human transformant transformed with the recombinant vector of claim 7.

9. A method of producing the protein or the salt thereof of claim 1, which comprises culturing the transformant of claim 8 to allow the same to produce the protein of claim 1.

10. A method of screening a compound that alters the bindability of an androgen and the protein or the salt thereof of claim 1, which comprises using the protein or the salt thereof of claim 1.

11. A kit for screening a compound that alters the bindability of an androgen and the protein or the salt thereof of claim 1, which comprises the protein or the salt thereof of claim 1.

12. A method of quantitating the mRNA that encodes the protein of claim 1, which comprises using the polynucleotide of claim 2.

13. A diagnostic method for transition of hormone-sensitive cancers to the androgen-independent stage, which comprises using the quantitation method of claim 12.

**Patentansprüche**

1. Protein, das die folgende Aminosäuresequenz (a) oder (b) umfasst:

(a) eine Aminosäuresequenz, in der Tryptophan bei Aminosäurenummer 746 durch Leucin in der Aminosäursequenz, die durch SEQ ID NR.: 2 dargestellt ist, ersetzt ist,
(b) eine Aminosäuresequenz, in der Tryptophan bei Aminosäurenummer 746 durch Leucin ersetzt ist und Threonin bei Aminosäurenummer 882 durch Alanin in der Aminosäuresequenz, die durch SEQ ID NR: 2 dargestellt ist, ersetzt ist, oder ein Salz davon.

2. Polynukleotid, das ein Polynukleotid umfasst, das das Protein nach Anspruch 1 kodiert.

3. Polynukleotid nach Anspruch 2, das eine DNA ist.

4. Polynukleotid nach Anspruch 2, das die folgende Basensequenz (a) oder (b) aufweist:

(a) eine Basensequenz, in der die Base bei Basennummer 2237 durch Thymin in der Basensequenz, die durch SEQ ID NR.: 1 dargestellt ist, ersetzt ist,
(b) eine Basensequenz, in der die Base bei Basennummer 2237 durch Thymin ersetzt ist und die Base bei Basennummer 2644 durch Guanin in der Basensequenz, die durch SEQ ID NR.: 1 dargestellt ist, ersetzt ist.

5. Diagnostisches Mittel, das das Polynukleotid nach Anspruch 2 umfasst.

6. Diagnostisches Mittel nach Anspruch 5, das für die Diagnose von hormonunabhängigen Krebsarten ist.

7. Rekombinanter Vektor, der das Polynukleotid nach Anspruch 2 umfasst.

8. Nichthumane Transformante, die mit dem rekombinanten Vektor nach Anspruch 7 transformiert ist.

9. Verfahren zur Herstellung des Proteins oder des Salzes davon nach Anspruch 1, welches Kultivieren der Transformante nach Anspruch 8 umfasst, um derselben zu erlauben, das Protein nach Anspruch 1 herzustellen.

10. Verfahren zum Screenen einer Verbindung, die die Bindungsfähigkeit eines Androgens und des Proteins oder des Salzes davon nach Anspruch 1 ändert, welches Verwenden des Proteins oder des Salzes davon nach Anspruch 1 umfasst.

11. Kit zum Screenen einer Verbindung, die die Bindungsfähigkeit eines Androgens und des Proteins oder des Salzes davon nach Anspruch 1 ändert, welches das Protein oder das Salz nach Anspruch 1 umfasst.

12. Verfahren zur Quantifizierung der mRNA, die das Protein nach Anspruch 1 kodiert, welches Verwenden des Polynukleotids nach Anspruch 2 umfasst.

13. Diagnostisches Verfahren für den Übergang von hormonsensitiven Krebsarten zu dem androgenunabhängigen Stadium, welches Verwenden des Quantifizierungsverfahrens nach Anspruch 12 umfasst.

**Revendications**

1. Protéine comprenant la séquence d'acides aminés (a) ou (b) indiquée ci-dessous :

a) la séquence d'acides aminés présentée en tant que Séquence N° 2, dans laquelle le résidu de trytophane n° 746 a été remplacé par un résidu de leucine,

b) la séquence d'acides aminés présentée en tant que Séquence N° 2, dans laquelle le résidu de trytophane n° 746 a été remplacé par un résidu de leucine et le résidu de thréonine n° 882 a été remplacé par un résidu d'alanine,

ou sel d'une telle protéine.

2. Polynucléotide comprenant un polynucléotide qui code une protéine conforme à la revendication 1.

3. Polynucléotide conforme à la revendication 2, qui est un ADN.

4. Polynucléotide conforme à la revendication 2, qui comporte la séquence de bases (a) ou (b) indiquée ci-dessous :

a) la séquence de bases présentée en tant que Séquence N° 1, dans laquelle la base n° 2237 a été remplacée par une thymine,
b) la séquence de bases présentée en tant que Séquence N° 1, dans laquelle la base n° 2237 a été remplacée par une thymine et la base n° 2644 a été remplacée par une guanine.

5. Agent de diagnostic, comprenant un polynucléotide conforme à la revendication 2.

6. Agent de diagnostic, conforme à la revendication 5, qui est conçu pour le diagnostic de cancers hormono-indépendants.

7. Vecteur recombiné, comprenant un polynucléotide conforme à la revendication 2.

8. Organisme non-humain transformé avec un vecteur recombiné conforme à la revendication 7.

9. Procédé de production d'une protéine conforme à la revendication 1 ou d'un sel d'une telle protéine, lequel procédé comporte le fait de cultiver un organisme transformé conforme à la revendication 8 de manière à lui permettre de produire une protéine conforme à la revendication 1.

10. Procédé de recherche par criblage d'un composé qui altère la capacité de liaison d'un androgène et d'une protéine conforme à la revendication 1 ou d'un sel d'une telle protéine, lequel procédé comporte le fait d'utiliser une protéine conforme à la revendication 1 ou un sel d'une telle protéine.

11. Trousse pour recherche par criblage d'un composé qui altère la capacité de liaison d'un androgène et d'une protéine conforme à la revendication 1 ou d'un sel d'une telle protéine, laquelle trousse comprend une protéine conforme à la revendication 1 ou un sel d'une telle protéine.

12. Procédé de dosage de l'ARNm codant une protéine conforme à la revendication 1, lequel procédé comporte le fait d'utiliser un polynucléotide conforme à la revendication 2.

13. Procédé permettant de diagnostiquer la transition d'un cancer hormono-sensible à un cancer androgéno-indépendant, lequel procédé comporte le fait d'avoir recours à un procédé de dosage conforme à la revendication 12.

# FIG. 1

# FIG. 2

# FIG. 3

# FIG. 4

# FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002162206 A

- JP 2002255612 A

### Non-patent literature cited in the description

- **Taplin et al.** *Cancer Res.,* 1999, vol. 59, 2511-2515
- **Veldscholte et al.** *Biochem. Biophys. Res. Commun.,* 1990, vol. 173, 534-540
- **Haapala et al.** *Lab. Invest.,* 2001, vol. 81, 1647-1651
- *Proc. Natl. Acad. Sci. USA,* 1988, vol. 85, 7211-7215
- **M. Bodanszky ; M.A. Ondetti.** Peptide Synthesis. Interscience Publishers, 1966
- **Schroeder ; Luebke.** The Peptide. Academic Press, 1965
- **Nobuo Izumiya et al.** Peptide Gosei-no-Kiso to Jikken. Maruzen Co, 1975
- **Haruaki Yajima ; Shunpei Sakakibara.** Seikagaku Jikken Koza. *Tanpakushitsu no Kagaku,* 1977, vol. IV, 205
- Zoku Iyakuhin no Kaihatsu. Peptide Synthesis. Hirokawa Shoten, vol. 14
- New PCR and its application. *Jikken Igaku,* 1997, vol. 15 (7
- **J. Sambrook et al.** Molecular Cloning. Cold Spring Harbor Lab. Press, 1989
- *Proc. Natl. Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **Vaughn, J. L. et al.** *In Vivo,* 1977, vol. 13, 213-217

- **Maeda et al.** *Nature,* 1985, vol. 315, 592
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- *Bio/Technology,* 1988, vol. 6, 47-55
- Saibo Kogaku. Shin Saibo Kogaku Jikken Protocol. Shujunsha, 1995, vol. 8, 263-267
- *Virology,* 1973, vol. 52, 456
- **Miller.** Journal of Experiments in Molecular Genetics. Cold Spring Harbor Laboratory, 1972, 431-433
- **Bostian, K. L. et al.** *Proc. Natl. Acad. Sci. U.S.A.,* 1980, vol. 77, 4505
- **Bitter, G. A. et al.** *Proc. Natl. Acad. Sci. U.S.A.,* 1984, vol. 81, 5330
- **Grace, T. C. C.** *Nature,* 1962, vol. 195, 788
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- *Genomics,* 1989, vol. 5, 874-879
- *Proceedings of the National Academy of Sciences of the United States of America,* 1989, vol. 86, 2766-2770
- **Nambi, P. et al.** *The Journal of Biological Chemistry,* 1992, vol. 267, 19555-19559

SEQUENCE LISTING

 <110> Takeda Chemical Industries, Ltd.

 <120> Mutated Androgen Receptor, Cancer Cells Expressing Same, Production Method Thereof and Use Thereof

 <130> 3056WOOP

 <150> JP 2002-162206
 <151> 2002-06-03

 <150> JP 2002-255612

<151> 2002-08-30

<160> 6

<170> PatentIn version 3.1

<210> 1
<211> 2775
<212> DNA
<213> Homo sapiens

<220>
<221> CDS
<222> (1).. (2775)
<223>

<400> 1

```
atg gaa gtg cag tta ggg ctg gga agg gtc tac cct cgg ccg ccg tcc      48
Met Glu Val Gln Leu Gly Leu Gly Arg Val Tyr Pro Arg Pro Pro Ser
1               5                   10                  15
aag acc tac cga gga gct ttc cag aat ctg ttc cag agc gtg cgc gaa      96
Lys Thr Tyr Arg Gly Ala Phe Gln Asn Leu Phe Gln Ser Val Arg Glu
                20                  25                  30
gtg atc cag aac ccg ggc ccc agg cac cca gag gcc gcg agc gca gca     144
Val Ile Gln Asn Pro Gly Pro Arg His Pro Glu Ala Ala Ser Ala Ala
                35                  40                  45
```

```
cct ccc ggc gcc agt ttg ctg ctg ctg cag cag cag cag cag cag cag        192
Pro Pro Gly Ala Ser Leu Leu Leu Leu Gln Gln Gln Gln Gln Gln Gln
        50              55              60

cag cag cag cag cag cag cag cag cag cag cag cag cag cag cag cag        240
Gln Gln Gln Gln Gln Gln Gln Gln Gln Gln Gln Gln Gln Gln Gln Gln
65              70              75              80

cag cag cag caa gag act agc ccc agg cag cag cag cag cag cag ggt        288
Gln Gln Gln Gln Glu Thr Ser Pro Arg Gln Gln Gln Gln Gln Gln Gly
                85              90              95

gag gat ggt tct ccc caa gcc cat cgt aga ggc ccc aca ggc tac ctg        336
Glu Asp Gly Ser Pro Gln Ala His Arg Arg Gly Pro Thr Gly Tyr Leu
                100             105             110

gtc ctg gat gag gaa cag caa cct tca cag ccg cag tcg gcc ctg gag        384
Val Leu Asp Glu Glu Gln Gln Pro Ser Gln Pro Gln Ser Ala Leu Glu
                115             120             125

tgc cac ccc gag aga ggt tgc gtc cca gag cct gga gcc gcc gtg gcc        432
Cys His Pro Glu Arg Gly Cys Val Pro Glu Pro Gly Ala Ala Val Ala
        130             135             140

gcc agc aag ggg ctg ccg cag cag ctg cca gca cct ccg gac gag gat        480
Ala Ser Lys Gly Leu Pro Gln Gln Leu Pro Ala Pro Pro Asp Glu Asp
145             150             155             160

gac tca gct gcc cca tcc acg ttg tcc ctg ctg ggc ccc act ttc ccc        528
Asp Ser Ala Ala Pro Ser Thr Leu Ser Leu Leu Gly Pro Thr Phe Pro
                165             170             175

ggc tta agc agc tgc tcc gct gac ctt aaa gac atc ctg agc gag gcc        576
Gly Leu Ser Ser Cys Ser Ala Asp Leu Lys Asp Ile Leu Ser Glu Ala
                180             185             190

agc acc atg caa ctc ctt cag caa cag cag cag gaa gca gta tcc gaa        624
Ser Thr Met Gln Leu Leu Gln Gln Gln Gln Gln Glu Ala Val Ser Glu
                195             200             205

ggc agc agc agc ggg aga gcg agg gag gcc tcg ggg gct ccc act tcc        672
Gly Ser Ser Ser Gly Arg Ala Arg Glu Ala Ser Gly Ala Pro Thr Ser
        210             215             220

tcc aag gac aat tac tta ggg ggc act tcg acc att tct gac aac gcc        720
Ser Lys Asp Asn Tyr Leu Gly Gly Thr Ser Thr Ile Ser Asp Asn Ala
225             230             235             240

aag gag ttg tgt aag gca gtg tcg gtg tcc atg ggc ctg ggt gtg gag        768
Lys Glu Leu Cys Lys Ala Val Ser Val Ser Met Gly Leu Gly Val Glu
                245             250             255
```

```
gcg ttg gag cat ctg agt cca ggg gaa cag ctt cgg ggg gat tgc atg     816
Ala Leu Glu His Leu Ser Pro Gly Glu Gln Leu Arg Gly Asp Cys Met
            260             265             270

tac gcc cca ctt ttg gga gtt cca ccc gct gtg cgt ccc act cct tgt     864
Tyr Ala Pro Leu Leu Gly Val Pro Pro Ala Val Arg Pro Thr Pro Cys
            275             280             285

gcc cca ttg gcc gaa tgc aaa ggt tct ctg cta gac gac agc gca ggc     912
Ala Pro Leu Ala Glu Cys Lys Gly Ser Leu Leu Asp Asp Ser Ala Gly
        290             295             300

aag agc act gaa gat act gct gag tat tcc cct ttc aag gga ggt tac     960
Lys Ser Thr Glu Asp Thr Ala Glu Tyr Ser Pro Phe Lys Gly Gly Tyr
305             310             315             320

acc aaa ggg cta gaa ggc gag agc cta ggc tgc tct ggc agc gct gca    1008
Thr Lys Gly Leu Glu Gly Glu Ser Leu Gly Cys Ser Gly Ser Ala Ala
            325             330             335

gca ggg agc tcc ggg aca ctt gaa ctg ccg tct acc ctg tct ctc tac    1056
Ala Gly Ser Ser Gly Thr Leu Glu Leu Pro Ser Thr Leu Ser Leu Tyr
            340             345             350

aag tcc gga gca ctg gac gag gca gct gcg tac cag agt cgc gac tac    1104
Lys Ser Gly Ala Leu Asp Glu Ala Ala Ala Tyr Gln Ser Arg Asp Tyr
            355             360             365

tac aac ttt cca ctg gct ctg gcc gga ccg ccg ccc cct ccg ccg cct    1152
Tyr Asn Phe Pro Leu Ala Leu Ala Gly Pro Pro Pro Pro Pro Pro Pro
        370             375             380

ccc cat ccc cac gct cgc atc aag ctg gag aac ccg ctg gac tac ggc    1200
Pro His Pro His Ala Arg Ile Lys Leu Glu Asn Pro Leu Asp Tyr Gly
385             390             395             400

agc gcc tgg gcg gct gcg gcg gcg cag tgc cgc tat ggg gac ctg gcg    1248
Ser Ala Trp Ala Ala Ala Ala Ala Gln Cys Arg Tyr Gly Asp Leu Ala
            405             410             415

agc ctg cat ggc gcg ggt gca gcg gga ccc ggt tct ggg tca ccc tca    1296
Ser Leu His Gly Ala Gly Ala Ala Gly Pro Gly Ser Gly Ser Pro Ser
            420             425             430

gcc gcc gct tcc tca tcc tgg cac act ctc ttc aca gcc gaa gaa ggc    1344
Ala Ala Ala Ser Ser Ser Trp His Thr Leu Phe Thr Ala Glu Glu Gly
            435             440             445

cag ttg tat gga ccg tgt ggt ggt ggt ggg ggt ggt ggc ggc ggc ggc    1392
Gln Leu Tyr Gly Pro Cys Gly Gly Gly Gly Gly Gly Gly Gly Gly Gly
            450             455             460
```

```
ggc ggc ggc ggc ggc ggc ggc ggc ggc ggc ggc ggc ggc gag gcg gga      1440
Gly Gly Gly Gly Gly Gly Gly Gly Gly Gly Gly Gly Gly Glu Ala Gly
465                 470             475                 480

gct gta gcc ccc tac ggc tac act cgg ccc cct cag ggg ctg gcg ggc      1488
Ala Val Ala Pro Tyr Gly Tyr Thr Arg Pro Pro Gln Gly Leu Ala Gly
                    485             490                 495

cag gaa agc gac ttc acc gca cct gat gtg tgg tac cct ggc ggc atg      1536
Gln Glu Ser Asp Phe Thr Ala Pro Asp Val Trp Tyr Pro Gly Gly Met
                500             505             510

gtg agc aga gtg ccc tat ccc agt ccc act tgt gtc aaa agc gaa atg      1584
Val Ser Arg Val Pro Tyr Pro Ser Pro Thr Cys Val Lys Ser Glu Met
                515             520             525

ggc ccc tgg atg gat agc tac tcc gga cct tac ggg gac atg cgt ttg      1632
Gly Pro Trp Met Asp Ser Tyr Ser Gly Pro Tyr Gly Asp Met Arg Leu
                530             535             540

gag act gcc agg gac cat gtt ttg ccc att gac tat tac ttt cca ccc      1680
Glu Thr Ala Arg Asp His Val Leu Pro Ile Asp Tyr Tyr Phe Pro Pro
545             550             555             560

cag aag acc tgc ctg atc tgt gga gat gaa gct tct ggg tgt cac tat      1728
Gln Lys Thr Cys Leu Ile Cys Gly Asp Glu Ala Ser Gly Cys His Tyr
                565             570             575

gga gct ctc aca tgt gga agc tgc aag gtc ttc ttc aaa aga gcc gct      1776
Gly Ala Leu Thr Cys Gly Ser Cys Lys Val Phe Phe Lys Arg Ala Ala
                580             585             590

gaa ggg aaa cag aag tac ctg tgc gcc agc aga aat gat tgc act att      1824
Glu Gly Lys Gln Lys Tyr Leu Cys Ala Ser Arg Asn Asp Cys Thr Ile
                595             600             605

gat aaa ttc cga agg aaa aat tgt cca tct tgt cgt ctt cgg aaa tgt      1872
Asp Lys Phe Arg Arg Lys Asn Cys Pro Ser Cys Arg Leu Arg Lys Cys
                610             615             620

tat gaa gca ggg atg act ctg gga gcc cgg aag ctg aag aaa ctt ggt      1920
Tyr Glu Ala Gly Met Thr Leu Gly Ala Arg Lys Leu Lys Lys Leu Gly
625                 630             635                 640

aat ctg aaa cta cag gag gaa gga gag gct tcc agc acc acc agc ccc      1968
Asn Leu Lys Leu Gln Glu Glu Gly Glu Ala Ser Ser Thr Thr Ser Pro
                645             650             655

act gag gag aca acc cag aag ctg aca gtg tca cac att gaa ggc tat      2016
Thr Glu Glu Thr Thr Gln Lys Leu Thr Val Ser His Ile Glu Gly Tyr
                660             665             670
```

```
gaa tgt cag ccc atc ttt ctg aat gtc ctg gaa gcc att gag cca ggt      2064
Glu Cys Gln Pro Ile Phe Leu Asn Val Leu Glu Ala Ile Glu Pro Gly
        675             680             685

gta gtg tgt gct gga cac gac aac aac cag ccc gac tcc ttt gca gcc      2112
Val Val Cys Ala Gly His Asp Asn Asn Gln Pro Asp Ser Phe Ala Ala
    690             695             700

ttg ctc tct agc ctc aat gaa ctg gga gag aga cag ctt gta cac gtg      2160
Leu Leu Ser Ser Leu Asn Glu Leu Gly Glu Arg Gln Leu Val His Val
705             710             715             720

gtc aag tgg gcc aag gcc ttg cct ggc ttc cgc aac tta cac gtg gac      2208
Val Lys Trp Ala Lys Ala Leu Pro Gly Phe Arg Asn Leu His Val Asp
            725             730             735

gac cag atg gct gtc att cag tac tcc tgg atg ggg ctc atg gtg ttt      2256
Asp Gln Met Ala Val Ile Gln Tyr Ser Trp Met Gly Leu Met Val Phe
            740             745             750

gcc atg ggc tgg cga tcc ttc acc aat gtc aac tcc agg atg ctc tac      2304
Ala Met Gly Trp Arg Ser Phe Thr Asn Val Asn Ser Arg Met Leu Tyr
        755             760             765

ttc gcc cct gat ctg gtt ttc aat gag tac cgc atg cac aag tcc cgg      2352
Phe Ala Pro Asp Leu Val Phe Asn Glu Tyr Arg Met His Lys Ser Arg
        770             775             780

atg tac agc cag tgt gtc cga atg agg cac ctc tct caa gag ttt gga      2400
Met Tyr Ser Gln Cys Val Arg Met Arg His Leu Ser Gln Glu Phe Gly
785             790             795             800

tgg ctc caa atc acc ccc cag gaa ttc ctg tgc atg aaa gca ctg cta      2448
Trp Leu Gln Ile Thr Pro Gln Glu Phe Leu Cys Met Lys Ala Leu Leu
            805             810             815

ctc ttc agc att att cca gtg gat ggg ctg aaa aat caa aaa ttc ttt      2496
Leu Phe Ser Ile Ile Pro Val Asp Gly Leu Lys Asn Gln Lys Phe Phe
            820             825             830

gat gaa ctt cga atg aac tac atc aag gaa ctc gat cgt atc att gca      2544
Asp Glu Leu Arg Met Asn Tyr Ile Lys Glu Leu Asp Arg Ile Ile Ala
            835             840             845

tgc aaa aga aaa aat ccc aca tcc tgc tca aga cgc ttc tac cag ctc      2592
Cys Lys Arg Lys Asn Pro Thr Ser Cys Ser Arg Arg Phe Tyr Gln Leu
        850             855             860

acc aag ctc ctg gac tcc gtg cag cct att gcg aga gag ctg cat cag      2640
Thr Lys Leu Leu Asp Ser Val Gln Pro Ile Ala Arg Glu Leu His Gln
865             870             875             880
```

```
ttc act ttt gac ctg cta atc aag tca cac atg gtg agc gtg gac ttt        2688
Phe Thr Phe Asp Leu Leu Ile Lys Ser His Met Val Ser Val Asp Phe
                885                 890                 895

ccg gaa atg atg gca gag atc atc tct gtg caa gtg ccc aag atc ctt        2736
Pro Glu Met Met Ala Glu Ile Ile Ser Val Gln Val Pro Lys Ile Leu
                900                 905                 910

tct ggg aaa gtc aag ccc atc tat ttc cac acc cag tga                    2775
Ser Gly Lys Val Lys Pro Ile Tyr Phe His Thr Gln
                915                 920
```

<210> 2
<211> 924
<212> PRT
<213> Homo sapiens

<400> 2

```
Met Glu Val Gln Leu Gly Leu Gly Arg Val Tyr Pro Arg Pro Pro Ser
1               5                   10                  15
Lys Thr Tyr Arg Gly Ala Phe Gln Asn Leu Phe Gln Ser Val Arg Glu
                20                  25                  30
Val Ile Gln Asn Pro Gly Pro Arg His Pro Glu Ala Ala Ser Ala Ala
            35                  40                  45
Pro Pro Gly Ala Ser Leu Leu Leu Leu Gln Gln Gln Gln Gln Gln Gln
        50                  55                  60
Gln Gln Gln Gln Gln Gln Gln Gln Gln Gln Gln Gln Gln Gln Gln Gln
65                  70                  75                  80
Gln Gln Gln Gln Glu Thr Ser Pro Arg Gln Gln Gln Gln Gln Gln Gly
                85                  90                  95
Glu Asp Gly Ser Pro Gln Ala His Arg Arg Gly Pro Thr Gly Tyr Leu
                100                 105                 110
Val Leu Asp Glu Glu Gln Gln Pro Ser Gln Pro Gln Ser Ala Leu Glu
            115                 120                 125
Cys His Pro Glu Arg Gly Cys Val Pro Glu Pro Gly Ala Ala Val Ala
            130                 135                 140
Ala Ser Lys Gly Leu Pro Gln Gln Leu Pro Ala Pro Pro Asp Glu Asp
145                 150                 155                 160
Asp Ser Ala Ala Pro Ser Thr Leu Ser Leu Leu Gly Pro Thr Phe Pro
                165                 170                 175
```

26

```
Gly Leu Ser Ser Cys Ser Ala Asp Leu Lys Asp Ile Leu Ser Glu Ala
            180                 185                 190
Ser Thr Met Gln Leu Leu Gln Gln Gln Gln Gln Glu Ala Val Ser Glu
            195                 200                 205
Gly Ser Ser Ser Gly Arg Ala Arg Glu Ala Ser Gly Ala Pro Thr Ser
        210                 215                 220
Ser Lys Asp Asn Tyr Leu Gly Gly Thr Ser Thr Ile Ser Asp Asn Ala
225                 230                 235                 240
Lys Glu Leu Cys Lys Ala Val Ser Val Ser Met Gly Leu Gly Val Glu
                245                 250                 255
Ala Leu Glu His Leu Ser Pro Gly Glu Gln Leu Arg Gly Asp Cys Met
            260                 265                 270
Tyr Ala Pro Leu Leu Gly Val Pro Pro Ala Val Arg Pro Thr Pro Cys
            275                 280                 285
Ala Pro Leu Ala Glu Cys Lys Gly Ser Leu Leu Asp Asp Ser Ala Gly
        290                 295                 300
Lys Ser Thr Glu Asp Thr Ala Glu Tyr Ser Pro Phe Lys Gly Gly Tyr
305                 310                 315                 320
Thr Lys Gly Leu Glu Gly Glu Ser Leu Gly Cys Ser Gly Ser Ala Ala
            325                 330                 335
Ala Gly Ser Ser Gly Thr Leu Glu Leu Pro Ser Thr Leu Ser Leu Tyr
            340                 345                 350
Lys Ser Gly Ala Leu Asp Glu Ala Ala Ala Tyr Gln Ser Arg Asp Tyr
            355                 360                 365
Tyr Asn Phe Pro Leu Ala Leu Ala Gly Pro Pro Pro Pro Pro Pro Pro
        370                 375                 380
Pro His Pro His Ala Arg Ile Lys Leu Glu Asn Pro Leu Asp Tyr Gly
385                 390                 395                 400
Ser Ala Trp Ala Ala Ala Ala Ala Gln Cys Arg Tyr Gly Asp Leu Ala
            405                 410                 415
Ser Leu His Gly Ala Gly Ala Ala Gly Pro Gly Ser Gly Ser Pro Ser
            420                 425                 430
Ala Ala Ala Ser Ser Ser Trp His Thr Leu Phe Thr Ala Glu Glu Gly
            435                 440                 445
Gln Leu Tyr Gly Pro Cys Gly Gly Gly Gly Gly Gly Gly Gly Gly Gly
        450                 455                 460
Gly Gly Gly Gly Gly Gly Gly Gly Gly Gly Gly Gly Gly Glu Ala Gly
465                 470                 475                 480
Ala Val Ala Pro Tyr Gly Tyr Thr Arg Pro Pro Gln Gly Leu Ala Gly
```

```
                          485                  490                  495
       Gln Glu Ser Asp Phe Thr Ala Pro Asp Val Trp Tyr Pro Gly Gly Met
                      500                  505                  510
       Val Ser Arg Val Pro Tyr Pro Ser Pro Thr Cys Val Lys Ser Glu Met
                      515                  520                  525
       Gly Pro Trp Met Asp Ser Tyr Ser Gly Pro Tyr Gly Asp Met Arg Leu
                  530                  535                  540
       Glu Thr Ala Arg Asp His Val Leu Pro Ile Asp Tyr Tyr Phe Pro Pro
       545                  550                  555                  560
       Gln Lys Thr Cys Leu Ile Cys Gly Asp Glu Ala Ser Gly Cys His Tyr
                      565                  570                  575
       Gly Ala Leu Thr Cys Gly Ser Cys Lys Val Phe Phe Lys Arg Ala Ala
                      580                  585                  590
       Glu Gly Lys Gln Lys Tyr Leu Cys Ala Ser Arg Asn Asp Cys Thr Ile
                  595                  600                  605
       Asp Lys Phe Arg Arg Lys Asn Cys Pro Ser Cys Arg Leu Arg Lys Cys
                  610                  615                  620
       Tyr Glu Ala Gly Met Thr Leu Gly Ala Arg Lys Leu Lys Lys Leu Gly
       625                  630                  635                  640
       Asn Leu Lys Leu Gln Glu Glu Gly Glu Ala Ser Ser Thr Thr Ser Pro
                      645                  650                  655
       Thr Glu Glu Thr Thr Gln Lys Leu Thr Val Ser His Ile Glu Gly Tyr
                  660                  665                  670
       Glu Cys Gln Pro Ile Phe Leu Asn Val Leu Glu Ala Ile Glu Pro Gly
                  675                  680                  685
       Val Val Cys Ala Gly His Asp Asn Asn Gln Pro Asp Ser Phe Ala Ala
                  690                  695                  700
       Leu Leu Ser Ser Leu Asn Glu Leu Gly Glu Arg Gln Leu Val His Val
       705                  710                  715                  720
       Val Lys Trp Ala Lys Ala Leu Pro Gly Phe Arg Asn Leu His Val Asp
                      725                  730                  735
       Asp Gln Met Ala Val Ile Gln Tyr Ser Trp Met Gly Leu Met Val Phe
                  740                  745                  750
       Ala Met Gly Trp Arg Ser Phe Thr Asn Val Asn Ser Arg Met Leu Tyr
                  755                  760                  765
       Phe Ala Pro Asp Leu Val Phe Asn Glu Tyr Arg Met His Lys Ser Arg
                  770                  775                  780
       Met Tyr Ser Gln Cys Val Arg Met Arg His Leu Ser Gln Glu Phe Gly
       785                  790                  795                  800
```

28

```
Trp Leu Gln Ile Thr Pro Gln Glu Phe Leu Cys Met Lys Ala Leu Leu
                    805                 810                 815
Leu Phe Ser Ile Ile Pro Val Asp Gly Leu Lys Asn Gln Lys Phe Phe
                820                 825                 830
Asp Glu Leu Arg Met Asn Tyr Ile Lys Glu Leu Asp Arg Ile Ile Ala
            835                 840                 845
Cys Lys Arg Lys Asn Pro Thr Ser Cys Ser Arg Arg Phe Tyr Gln Leu
    850                 855                 860
Thr Lys Leu Leu Asp Ser Val Gln Pro Ile Ala Arg Glu Leu His Gln
865                 870                 875                 880
Phe Thr Phe Asp Leu Leu Ile Lys Ser His Met Val Ser Val Asp Phe
                885                 890                 895
Pro Glu Met Met Ala Glu Ile Ile Ser Val Gln Val Pro Lys Ile Leu
            900                 905                 910
Ser Gly Lys Val Lys Pro Ile Tyr Phe His Thr Gln
        915                 920
```

<210> 3
<211> 15
<212> DNA
<213> Homo sapiens

<220>
<221> misc_feature
<223> ARE sequence in human PSA promoter.

<400> 3
agaacagcaa gtgct          15

<210> 4
<211> 35
<212> DNA
<213> Homo sapiens

<220>
<221> misc_feature
<223> ARR sequence in human PSA promoter.

<400> 4
gtggtgcagg gatcagggag tctcacaatc tcctg          35

<210> 5
<211> 35
<212> DNA
<213> Artificial Sequence

<220>
<221> misc_feature

<223> Oligonucleotide designed to act as primer for amplifying human PSA promoter.

<400> 5
ggagctcgaa ttccacattg tttgctgcac gttgg          35

<210> 6
<211> 34
<212> DNA
<213> Artificial Sequence

<220>
<221> misc_feature
<223> Oligonucleotide designed to act as primer for amplifying human PSA promoter.

<400> 6
caagctttgg ggctggggag cctcccccag gagc          34